# EUROPEAN PATENT APPLICATION

(11) **EP 2 612 666 A2**
(43) Date of publication of application: **10.07.2013**
(21) Application number: 13150724.6
(22) Date of filing: 02.04.2007
(51) Int. Cl.: A61K 31/198, A61P 1/06

(54) **Treatments using citrulline**

(30) Priority: 04.04.2006 US 789330 P; 17.05.2006 US 747493 P
(62) Divisional of application: 07754640.6
(71) Applicant: Nestec S.A., 1800 Vevey (CH)
(72) Inventor: Deutz, Nicolaas Emile, 6215 Maastricht (NL); Greenberg, Norman, Alan, New Hope, MN Minnesota 55427 (US); Kaspar, Kala Marie, 1005 lausanne (CH); Kvamme, Candis, Brooklyn Park, MN Minnesota 55445 (US); Luiking, Yvette Charlotte, 6132 Sittard (NL)
(74) Representative: Chautard, Cécile

(57) **Abstract**

The invention provides a method and formulation for the treatment or maintenance of conditions that would be benefited from increasing or maintaining Arginine levels in the blood, and having improved taste characteristics over current Arginine supplementations. Further, this maintenance of Arginine levels in the blood will be beneficial in acute and chronic diseases with an impaired arginine to citrulline production rate. Further the invention provides a method for treating at least one of satiety and dyspepsia in an individual. In one embodiment, the method includes administering to an individual an effective amount of L-citrulline.

## Description

### BACKGROUND OF THE INVENTION

### 1. Technical Field

The invention relates to the treatment or maintenance of conditions that would be benefited from increasing or maintaining Arginine levels in the blood, and having improved taste characteristics over current Arginine supplementations. Further the invention relates generally to the treatment of early satiety, and more particularly, to the administration of L-citrulline in order to promote relaxation of smooth muscles of the stomach. Additionally, the invention relates generally the use of citrulline for the benefits of increased wound healing and improved microcirculation in renal impaired patients when administered before dialysis is initiated or while receiving dialysis or both.

### 2. Background Art

Early satiety is the feeling of fullness prior to the consumption of adequate calories and/or liquids. Dyspepsia is a disorder of the digestive system characterized by chronic or recurrent upper abdominal symptoms, including early satiety, discomfort, heartburn, and/or nausea following the ingestion of food. Acute or prolonged early satiety or dyspepsia may result in malnutrition and/or dehydration. Some individuals, such as cancer patients, those with chronic diseases such as human immunodeficiency virus (HIV), and the elderly, often experience early satiety and/or dyspepsia. These individuals are more likely, therefore, to experience insufficient caloric or liquid intake, which may further complicate existing health problems.

Known methods of treating early satiety and dyspepsia generally focus on either the stimulation of an individual's appetite or the administration of low-volume, nutrient- and calorie-rich dietary supplements. Neither method is satisfactory. Increasing the appetite of an individual experiencing early satiety or dyspepsia simply increases the likelihood that the individual will continue to eat after feeling full or the onset of dyspeptic symptoms, often causing great discomfort. Nutrient- and calorie-rich dietary supplements are generally not as flavorful as the individual's normal diet and are therefore less likely to be consumed in the quantities necessary to mitigate the malnourishing and/or dehydrating effects of early satiety or dyspepsia.

Muscles of the stomach provide feedback to the brain through the stimulation of stretch and nutrient receptors. When the stomach distends as a result of the ingestion of food, stretch receptors are activated in the stomach muscle, triggering inhibitory signals in the vagus nerve, resulting in a desire to stop eating. Such distention is particularly evident in the accommodation reflex of the fundus. If the stomach muscles relax, particularly those of the fundus, there is a reduction in satiety that permits an individual to consume more food before satiety is achieved. Thus, relaxation of the stomach smooth muscle prevents early satiety, increasing the amount of food present in the stomach prior to the activation of stretch receptors.

Other methods of treating early satiety have therefore focused on relaxation of stomach muscles. The biochemical mechanism by which stomach smooth muscles are relaxed is known. Nitric oxide (NO) binds to a cellular protein receptor (e.g., guanylyl cyclase) through interaction with either a metal ion in the protein or a sulfur atom in cysteine. The binding of NO causes a change in the protein, which in turn causes an increase in the concentration of the second messenger cyclic guanosine monophosphate (cGMP). cGMP causes relaxation of smooth muscle, such as that of the lower esophageal sphincter, fundus, pylorus, sphincter of Oddi, intestine, and anus. Thus, an increase in NO will lead to increased relaxation of smooth muscle, including the smooth muscles of the stomach, particularly the fundus.

The primary source for NO is the amino acid arginine. Arginine is metabolized into citrulline and NO via the enzyme nitric oxide synthase (NOS). Accordingly, a method of treating early satiety via relaxation of stomach muscles might include the increased consumption of arginine. The difficulty with such a method, however, is that only a portion of the arginine consumed by an individual remains available for metabolization to NO. As much as 60% of ingested arginine is metabolized in the liver by arginase before entering the circulation, where any remaining arginine may be metabolized to citrulline and NO. Accordingly, such a method would require the ingestion of a large quantity of an arginine-rich dietary supplement in order to induce a relaxation of stomach muscles. Such a large quantity of a dietary supplement would itself induce a satiating effect, countering any relaxing effect produced by subsequent NO production. An alternative source for arginine is the endogenous production of arginine from the amino acid citrulline. This route contributes about 20% to whole body arginine production. Citrulline is produced in the intestine and enters the circulation without being metabolized by the liver, with almost complete conversion to arginine in the kidneys.

To this extent, a need exists for a method for treating early satiety that does not suffer from the above defects.

Further, the use of some dietary amino acids, including L-arginine, in oral nutritional compositions have an objectionable taste at alkaline pH. In the case of L-arginine it is the direct result of its basic side chain. US Patent application 20020193342 Hamman & Calton (2002) 'Modifying undesirable tastes' describes the use of sucralose to mask flavor of free amino acid. US Patent 5780039 Greenberg et al. (1998) describes the improved taste of an oral nutritional composition arginine and encapsulation of fatty acids. US Patent application 20020068365 Kuhrts (2002) uses small coated granules of 80% L-arginine and 20% coating. The coating provided taste masking of the bitter L-arginine and produced a sustained-release, powdered drink mix. Prior methods, although somewhat effective in masking the flavors, the underlying problem of the taste of the L-arginine still exists.

### SUMMARY OF THE INVENTION

The invention provides a method for treating at least one of satiety and dyspepsia in an individual. In one embodiment, the method includes administering to the individual an effective amount of L-citrulline.

A first aspect of the invention provides a method for treating at least one of satiety and dyspepsia in an individual, the method comprising: increasing a concentration of nitric oxide (NO) in the individual's cells, wherein the increased concentration of NO results in a relaxation of smooth muscles of the stomach.

A second aspect of the invention provides an orally-administrable nutritional supplement comprising: a quantity of L-citrulline effective to cause an increase in a concentration of nitric oxide (NO) in the cells of an individual.

A third aspect of the invention is to improve the flavor of the compositions that include the basic amino acid L-arginine's bitter flavors. Typically the pH of a solution is balanced through the addition of acid, such as phosphoric acid, but this additional acid may increase problems in beverage stability. However, the complete or partial substitution with L-citrulline for L-arginine results in a more neutral pH beverage that does not require supplemental acid (e.g., phosphoric acid) to balance pH.

A forth aspect of the invention is an effective mechanism for increasing or maintaining Arginine levels in the blood, using an oral supplementation of L-Citrulline or L-Ornithine. Arginine has many effects in the body that include modulation of immune function, wound healing, hormone secretion, vascular tone, insulin sensitivity, and endothelial function. It is within the scope of this invention that the use of citrulline to maintain arginine levels would lead to similar benefits.

A fifth aspect of the invention is an effect mechanism for the treatment or prophylaxis of an acute or chronic disease in a mammal, especially a human being, that is characterized by a lower than normal citrulline to arginine conversion rate, comprising at least one of citrulline, or a precursor of citrulline, in a daily dose for said mammal of at least the amount by which the citrulline to arginine conversion rate is reduced.

A sixth aspect of the invention is the use of citrulline for the benefits of increased wound healing and improved microcirculation in renal impaired patients when administered before dialysis is initiated or while receiving dialysis or both. This is, at least partly, accomplished by decreased urea and/or ammonia production by the mammal.

The illustrative aspects of the present invention are designed to solve the problems herein described and other problems not discussed, which are discoverable by a skilled artisan.

### DETAILED DESCRIPTION

As indicated above, the invention provides a method for treating early satiety and/or dyspepsia in an individual. The invention further provides products for such treatment.

As used herein, the terms "treatment," "treating," and "treat" refer to both prophylactic or preventive treatment and curative or disease-modifying treatment, including treatment of patients at risk of contracting a disease or suspected to have contracted a disease, as well as patients who are ill or have been diagnosed as suffering from a disease or medical condition. The terms "treatment," "treating," and "treat" also refer to the maintenance and/or promotion of health in an individual not suffering from a disease but who may be susceptible to the development of an unhealthy condition, such as early satiety or dyspepsia. Consequently, an "effective amount" is an amount that treats a disease or medical condition in an individual or, more generally, provides a nutritional, physiological, or medical benefit to the individual.

As used herein, mammal includes but is not limited to rodents, aquatic mammals, domestic animals such as dogs and cats, farm animals such as sheep, pigs, cows and horses, and humans. Wherein the term mammal is used, it is contemplated that it also applies to other animals that are capable of the effect exhibited or intended to be exhibited by the mammal.

As described above, arginine, like most amino acids, is largely metabolized in the liver prior to entry into the circulatory system. This limits the usefulness of arginine as a source of nitric oxide (NO) for smooth muscle relaxation.

However, a few amino acids pass through the liver with little or no metabolization. One such amino acid is L-citrulline, a precursor of arginine. Just as arginine is converted to citrulline and NO, L-citrulline is converted to arginine in the mitochondria. The majority of circulating L-citrulline is converted in the kidneys, which are comprised of highly metabolically active tissue. As such, L-citrulline circulating in the bloodstream is first converted to arginine and then in cells to citrulline and NO.

Significantly, the conversion of L-citrulline to arginine occurs continuously, as long as L-citrulline is circulating in the bloodstream. As a result, circulating L-citrulline makes it possible to maintain elevated concentrations of arginine over time, which in turn makes it possible to maintain a steady release of NO in cells, causing a relaxation of stomach muscles and an abatement or avoidance of early satiety. At least some cases of dyspepsia are associated with the contraction of smooth muscles of the digestive system, particularly the smooth muscles of the stomach. Accordingly, the administration of L-citrulline may also be used to treat dyspepsia.

According to the invention, an effective amount of L-citrulline may be administered to an individual by any number of methods, as will be recognized by one having ordinary skill in the art. In one embodiment, an effective amount of L-citrulline is administered to an individual in an orally-administrable nutritional supplement. A supplement according to the invention may further contain any number of ingredients that provide a nutritional, physiological, or medical benefit to an individual. Such ingredients include, for example, proteins, soluble and/or insoluble fibers, fatty acids, vitamins, minerals, sugars and/or other carbohydrates, flavor agents, and medicaments or other therapeutic agents.

The use of some dietary amino acids, including L-arginine, in oral nutritional compositions have an objectionable taste at alkaline pH. In the case of L-arginine it is the direct result of its basic side chain. In order to improve the flavor of the oral composition the high/alkaline pH of the composition is neutralized through addition of acid (low pH). The higher acidity also improves the flavor of these compositions by reducing the bitter flavors associated with the basic amino acid (e.g., L-arginine).

Additional complications arise when acids are used in nutritional compositions. For example, phosphoric acid is often used, but results in elevated phosphorus content of the composition. The amino acid L-citrulline is a precursor to the L-arginine. As a result, we propose that L-citrulline can substitute for L-arginine in nutritional compositions. Unlike arginine, citrulline is not metabolized by the liver following entry into the bloodstream through absorption from the diet or *de novo* intestinal production. Citrulline is enzymatically converted to arginine by mitochondria via a part of the urea cycle.

Because the use of citrulline eliminates the need to use acid to neutralize pH and offset the bitter flavors caused by the basic amino acid L-arginine, it is easier to include dietary fats into a nutritional composition. It is a substantial challenge to add dietary fats to compositions with very high or low pH. As a result, it is proposed that citrulline is a viable alternative to arginine in a dietary composition as it increases options in formulation. As a result, benefits of the use of citrulline include reduction of acids and the ability to incorporate dietary lipids into the composition to improve the nutritional value of the composition.

It is a substantial challenge to add dietary fats to compositions with very high or low pH. Liquid nutritional compositions comprising L-arginine required inclusion of acid to offset the bitter flavor imparted by the alkaline pH of L-arginine. Substitution of L-arginine with L-citrulline, either completely or in part, eliminates or reduces the need for large amounts of acid in the composition, respectively.

In a further aspect of the invention, is a composition suitable for the treatment or prophylaxis of an acute or chronic disease in a mammal, especially a human being, that is characterized by a lower than normal citrulline to arginine conversion rate, comprising at least one of citrulline, or a precursor of citrulline, in a daily dose for said mammal of at least the amount by which the citrulline to arginine conversion rate is reduced.

Arginine deficiency can occur within hours of surgery or other trauma as a result of physiological changes to the insult inflicted on the body. Deficiency can also occur during sepsis. Arginine deficiency is, at least partly, caused by increased arginase enzyme activity, therefore increased arginine activity.

Citrulline can be given via the GI tract in oral and enteral products. Citrulline, endogenously, is a product of the metabolism of glutamine in the gut, generated from ornithine as part of the urea cycle, and formed by nitric oxide synthases distributed in the body. Arginine is generated from citrulline, mainly in the kidney, through its metabolism by argininosuccinate synthase (EC 6.3.4.5) and argininosuccinate lyase (EC 4.3.2.1).

Septic patients have been considered arginine deficient as a result of decreased production of arginine from citrulline and also inadequate dietary intake. This has resulted in the use of arginine enriched enteral formulas. Arginine is the precursor for nitric oxide, a known vasodilator. Enhanced nitric oxide production due to arginine infusion has been considered detrimental because of anticipated hemodynamic instability and nitrosylation of proteins. Therefore, these effects were studied during prolonged continuous intravenous arginine supplementation as a single component in patients with severe sepsis.

### Example 1:

Methods: According to a placebo-controlled double-blind study design, ICU patients with severe sepsis/septic shock (<48h; APACHE II scores between 18-41) were randomized in a group treated with intravenous 72-h L-arginine-HCl (1.2 µmol/kg.min, n=9) or isocaloric L-alanine (placebo; n=9). All patients were treated with norepinephrine in a range between 0.08-0.9 g/kg.min at the start of study. Haemodynamics were recorded at 2-h intervals throughout the protocol. Blood was sampled at baseline and at 24-h intervals and analysed for plasma nitrotyrosine levels. Repeated measurements ANOVA was used; data are means±SEM. Results: Plasma nitrotyrosine did not differ between patients treated with arginine or placebo (for comparison, normal values are about 1 nM). No significant differences in systemic blood pressure and norepinephrine dose between both groups were observed.

| | Group | Baseline | 24 | 48 | 72h | P |
|---|---|---|---|---|---|---|
| Plasma nitrotyrosine *(nM)* | PLAC | 0.45±0.04 | 0.44±0.05 | 3.15±2.4 | 9.57±8.39 | |
| | ARG | 0.54±0.07 | 0.55±0.10 | 1.20±0.49 | 2.11±1.31 | |
| MAP *(mmHg)* | PLAC | 78±3 | 79±4 | 82±4 | 86±4 | T (0.09) |
| | ARG | 82±4 | 81±3 | 85±4 | 84±5 | |

| | | | | | | |
|---|---|---|---|---|---|---|
| PLAC, placebo; ARG, Arginine treated. Repeated measurements ANOVA: T, time effect. | | | | | | |

**Conclusions:** Arginine infusion does not enhance the oxidative effect on plasma proteins and does not affect systemic blood pressure. Therefore detrimental effects of arginine on oxidative stress or the haemodynamic condition in severe septic patients are not present. Therefore, Citrulline administration leads to correction of the arginine deficiency, in some cases possibly raising the arginine levels over established normal levels, this elevation is theorized to be the body's way of meeting an excess demand in some physiologically stressful situations. These levels are believed to be safe since the body is not believed to convert citrulline in excess to arginine.

Recent studies have shown that the level of arginine in hemodialysis patients is elevated both pre and post dialysis run (Chuang et al., Clinica Chimica Acta 364; 216, 2006). Citrulline is also elevated both pre and post dialysis run. However, the arginine level was decreased by 36% while the citrulline level was decreased by 258% during the same dialysis treatment. This larger reduction in citrulline brings up the possibility of using citrulline as a substitute for arginine for dialysis patients that are in need of improved wound healing due to diabetes and lack of mobility.

Excessive arginine has been shown to cause additional renal problems in patients with compromised kidneys (Efron and Barbul, J. Renal Nutr. 9(3)142, 1999). Citrulline can be used to supply arginine via conversion in the body. Additionally, citrulline has one less amino group than arginine. This reduces the amount of ammonia and urea produced and decreases the stress on the already compromised kidney.

Thus, the use of citrulline in place of arginine could allow for the increased benefits of wound healing and microcirculation in renal impaired patients both before dialysis is initiated and while receiving dialysis.

In a further aspect of this invention, arginine has many effects in the body that include modulation of immune function, wound healing, hormone secretion, vascular tone, insulin sensitivity, and endothelial function. It is within the scope of this invention that the use of citrulline to maintain arginine levels would lead to similar benefits.

The foregoing description of various aspects of the invention has been presented for purposes of illustration and description. It is not intended to be exhaustive or to limit the invention to the precise form disclosed, and obviously, many modifications and variations are possible. Such modifications and variations that may be apparent to a person skilled in the art are intended to be included within the scope of the invention as defined by the accompanying claims.

## Claims

1. A composition containing containing L-citrulline or L-ornithine, a precursor or salt of L-citrulline or L-ornithine or a combination thereof for use in the treatment or modulation of immune function, wound healing, hormone secretion, vascular tone, insulin sensitivity, and endothelial function in a mammal.

2. The composition according to claim 1, wherein the mammal is a human.

3. The composition according to claim 1, wherein said L-citrulline or L-ornithine, a precursor or salt of L-citrulline or L-ornithine or a combination thereof is modulating insulin sensitivity.

4. The composition according to claim 1, wherein at least a portion of the quantity of L-citrulline or L-ornithine, a precursor or salt of L-citrulline or L-ornithine or a combinations thereof is metabolized to form arginine.

5. The composition according to claim 1, wherein said mammal is renally impaired, optionally wherein said mammal is administered said composition before dialysis is initiated or while receiving dialysis, or before dialysis is initiated and while receiving dialysis.

6. The composition according to claim 5, wherein the amount of ammonia or urea or ammonia and urea produced by the mammal is decreased.

7. The composition according to claim 5, wherein the stress on the kidney is decreased.

8. A composition containing L-citrulline or L-ornithine, a precursor or salt of L-citrulline or L-ornithine or a combinations thereof for use in the treatment or prophylaxis of an acute or chronic disease that is **characterized by** a lower than normal citrulline to arginine conversion rate.

9. The composition according to claim 8, wherein an amount of containing L-citrulline or L-ornithine, a precursor or salt of L-citrulline or L-ornithine or a combinations thereof is at least the amount by which the citrulline to arginine conversion rate is reduced.

10. The composition according to claim 8, wherein the composition is administered to a mammal, optionally wherein the mammal is a human.

11. A composition comprising citrulline or a precursor of citrulline for use in the treatment or prophylaxis of an acute or chronic disease in a mammal, wherein the disease is **characterized by** a lower than normal citrulline to arginine conversion rate and the composition comprises citrulline or a precursor of citrulline in an amount by which the citrulline to arginine conversion rate is reduced.

12. The composition of claim 10, wherein
(a) the mammal is a human; optionally wherein the quantity of L-citrulline is effective to cause an increase in the concentration of NO in the individual's cells; or
(b) at least a portion of the quantity of L-citrulline is metabolized to form arginine;
(c) at least a portion of the arginine is metabolized to form citrulline and NO; or
(d) the quantity of L-citrulline is included in an orally-administrable nutritional supplement; or
(e) the supplement further includes at least one of the following: a protein, a soluble fiber, an insoluble fiber, a fatty acid, a vitamin, a mineral, a carbohydrate, a flavour agent, a medicament, and a therapeutic agent.

13. A method of improving the taste, flavor, or nutritional value of a supplement for increasing or maintaining blood arginine levels, the method comprising: administering to a composition containing L-citrulline or L-ornithine, a precursor or salt of L-citrulline or L-ornithine, or a combination thereof.

14. The method of claim 12 wherein
(a) the taste is improved through the neutralization of the alkaline pH of a composition containing L-arginine through the substitution of L-arginine with L-citrulline; or
(b) the improved taste is a reduction in bitterness or
(c) the improved taste is due to the addition of dietary fats.
